Europäisches Patentamt

European Patent Office

Office européen des brevets

(11) Publication number: **0 167 852 B1**

# EUROPEAN PATENT SPECIFICATION

(45) Date of publication of patent specification: **02.09.92**    (51) Int. Cl.5: **C12N 15/23**, C12N 1/20, C12N 5/00, C12N 15/85

(21) Application number: **85107142.3**

(22) Date of filing: **11.06.85**

The file contains technical information submitted after the application was filed and not included in this specification

(54) **Human interferon-gamma.**

(30) Priority: **11.06.84 JP 119648/84**
**27.04.85 JP 91618/85**
**27.04.85 JP 91619/85**

(43) Date of publication of application:
**15.01.86 Bulletin 86/03**

(45) Publication of the grant of the patent:
**02.09.92 Bulletin 92/36**

(84) Designated Contracting States:
**BE CH DE FR GB IT LI NL SE**

(56) References cited:
**EP-A- 0 063 482**
**EP-A- 0 077 670**
**EP-A- 0 088 540**
**EP-A- 0 159 714**
**DD-A- 206 791**

(73) Proprietor: **KANEGAFUCHI KAGAKU KOGYO KABUSHIKI KAISHA**
**2-4 Nakanoshima 3-chome**
**Kita-ku Osaka-shi Osaka-fu 530(JP)**

(72) Inventor: **Kakutani, Tetsu**
**835-1, Befu, Befu-cho**
**Kakogawa-shi Hyogo-ken(JP)**
Inventor: **Matsumoto, Keiji**
**20-16-204, Tendo-cho**
**Nishinomiya-shi Hyogo-ken(JP)**
Inventor: **Maruyama, Hiroyuki**
**2-63, Okihama-cho Takasago-cho**
**Takasago-shi Hyogo-ken(JP)**
Inventor: **Nakagawa, Kaku**
**2-63, Okihama-cho Takasago-cho**
**Takasago-shi Hyogo-ken(JP)**
Inventor: **Yokota, Shinichi**
**12-3, Nishishinmachi 2-chome**
**Akashi-shi Hyogo-ken(JP)**
Inventor: **Niwa, Hideo**
**2-63, Okihama-cho Takasago-cho**
**Takasago-shi Hyogo-ken(JP)**

CHEMICAL ABSTRACTS, vol. 97, no. 9, 30th August 1982, page 153, abstract no. 67176h, Columbus, Ohio, US; M.F. LAW et al.: "Expression of selective traits in mouse cells transformed with a BPV DNA-derived hybrid molecule containing Escherichia coli gpt.", & EUKARYOTIC VIRAL VECTORS, [CONF.], 1981 (PUB. 182), 79-85

THE EMBO JOURNAL, vol. 1, no. 8, 1982, pages 953-958, IRL Press Ltd, Oxford, GB; Y. TAYA et al.: "Cloning and structure of the human immune interferon-gamma chromosomal gene"

PHIL. TRANS. R. SOC. LOND. vol. B 299, 1982, pages 29-38, GB; W. FIERS et al.: "The human fibroblast and human immune interferon genes and their expression in homologous and heterologous cells"

PROCEEDINGS OF THE NATIONAL ACADEMY OF SCIENCES OF THE USA, vol. 81, no. 16, August 1984, pages 5086-5090, Washington, US; R. FUKUNAGA et al.: "Constitutive production of human interferons by mouse cells with bovine papillomavirus as a vector"

Inventor: **Shinjo, Katsuhiro**
**2-63, Okihama-cho Takasago-cho**
**Takasago-shi Hyogo-ken(JP)**
Inventor: **Kawaharada, Hajime**
**2183-4, Shinzaike Hiraoka-cho**
**Kakogawa-shi Hyogo-ken(JP)**
Inventor: **Watanabe, Kiyoshi**
**15-41, Matsugaoka 5-chome**
**Akashi-shi Hyogo-ken(JP)**

(74) Representative: **Vossius & Partner**
**Siebertstrasse 4 P.O. Box 86 07 67**
**W-8000 München 86(DE)**

## Description

The present invention relates to the chromosomal DNA sequence coding for human interferon-γ - (hereinafter referred to as HuIFN-γ) which is ligated to a promoter sequence that enables the IFN-γ to be expressed in animal cell cultures, to cell cultures transformed with said DNA sequence, and to a method for the production of HuIFN-γ using said transformed cell cultures.

Interferon (hereinafter referred to as IFN) was, at first, discovered as a virus-inhibiting substance, but thereafter was shown to be a substance that bears various biological and immunological activities. From fairly long ago, IFN was known to be effective in inhibiting cell proliferation [Rubin, B.Y. et al (1980): Proc. Natl. Acad. Sci. USA, 77, 5928]. Further with the recent advances in immunological science, it was found that IFN can activate natural killer cells and other cells having antibody-dependent cell cytotoxicity which are supposed to take part in what is called immunological surveillance mechanism of cancer, so as to enhance the antitumor activity of those cells [Catalona, W.J. et al. (1981): Nature, 291, 77]. It has been also shown that IFN enhances the activity of cytotoxic T-cells [Lindahl,P.et al. (1972): Proc.Natl.Acad.Sci.,69, 721], and activates macrophages to change them to ones with antitumor activities [Le,J.et al. (1983): J.Immunol.,131,2821]. These findings indicate that IFN potentially is an antitumor agent.

According to differences in physiological,biochemical and immunological properties of the proteins, or the producing cells and in the induction mechanisms, HuIFNs are classified into three groups, which are called IFN-α, IFN-β, and IFN-γ,respectively[Stewart II,W.E. et al. (1980): Nature,286,110].

Among others, IFN-γ has the ability to inhibit cell proliferation at much lower concentrations than IFN-α and IFN-β [Rubin,B.Y.et al.(1980): Proc.Natl.Acad.Sci. USA,77, 5928)]. It also activates cells,including natural killer cells, killer T-cells, K-cells and macrophages which participate in what is called immunological surveillance mechanism of cancer. Therefore great expectations are directed to the clinical application of IFN-γ.

HuIFN-γ is known to be induced in human lymphocytes stimulated by phytohemaagglutinin,staphylococcal enterotoxin A,concanavalin A,or galactose oxidase (Wheelock,E.F. (1965): Science,149,310; Langford,M.P. et al. (1979): Infect.Immun., 26, 36; de Lay,M.etal. (1980): Eur.J.Immunol.,10,877; Dianzani,F. et al.(1979): Infect.Immun.,25,879]. Nevertheless,since in these production methods large amounts of fresh human lymphocytes are needed, the mass production of HuIFN-γ as a therapeutic drug by such means is rather difficult. Recently the cloning of cDNA has made possible the production of HUIFN-γ-like proteins by Escherichia coli [Gray, P.W. et al. (1982): Nature, 295, 503]. However, since the mechanisms of protein synthesis in animal cells are different from those of microorganisms, IFN-γ produced by microorganisms is often different from those naturally produced with respect to the terminal amino groups of protein. Furthermore, IFN-γ produced by microorganisms has no combined sugar chains, while the natural HuIFN-γ has them. Thus IFN-γ produced by a microbial protein synthesis system is substantially different from the natural HuIFN-γ, and therefore, in cases of prolonged or frequent use of such an IFN-γ, problems as the reduction of activities of the drug by antigen-antibody reaction and allergic responses such as shock reactions have to be taken into account According to Le et al., IFN-γ produced in Escherichia coli is different from natural HuIFN-γ in antigen-antibody. reactions with monoclonal antibodies, and the difference of reactivity is not due to the absence of sugar chains[Le et al. (1984): J. Immunol., 132, 1300].

HuIFN-γ was also produced by cloned T-cell lines [Nathan, H. et al. (1981): Nature, 292, 842], T-cell hybridomas [Le et al. (1982): Proc. Natl. Acad. Sci. USA, 79, 7857], and T-cells which were transformed by adult T-cell leukemia virus [Sugamura, K. et al. (1983): J. Immunol., 131, 1611]. Since these cells are supposed to contain human leukemia virus as provirus, or to release virus particles, their use in the production of IFN-γ is problematic with respect to biohazards [Sugamura, K. et al. (1983): ibid.].

On the other hand, production of HuIFN-γ was also attempted using animal cell cultures in which the simian virus 40 (hereinafter referred to as SV40) promoter sequence had been ligated to the IFN-γDNA sequence [Gray, P.W. et al. (1982): Nature, 295, 503; Haynes, J. et al. (1983): Nucleic Acids Res., 11, 687; Scahill, S,J, et al (1983); Proc. Natl. Acad. Sci. USA, 80, 4654; Devos, R. et al. (1982); Nucleic Acids Res., 10, 2487' European Patent Application No. 0077670; European Patent Application No. 0088540]. But DNA used in these production methods was cDNA.

It has been known that many proteins of higher organisms are encoded by chromosomal DNA sequences in the form of splitted sequences. The DNA sequences which encode mature messenger RNA are called exons, and the intervening sequences are introns. Although there are still unclear points as to the biological significance and function of intron, it has been known that far less ovalbumin [Wickens, M.P. et al (1980): Nature, 285, 628], or viral protein [Lai, C.J. et al. (1979): Proc. Natl. Acad. Sci. USA. 76, 71] is produced in animal cell cultures, into which an intron- free ovalbumin gene or viral gene is introduced, than in animal cell cultures transformed with intron-containing sequences. Further, it is known that the addition of introns of the β-globin gene to an intron-free SV40 gene causes the accumulation of stable messenger RNA (hereinafter referred to as mRNA) [Homer, D.H. et al. (1979): Cell, 18, 1299).

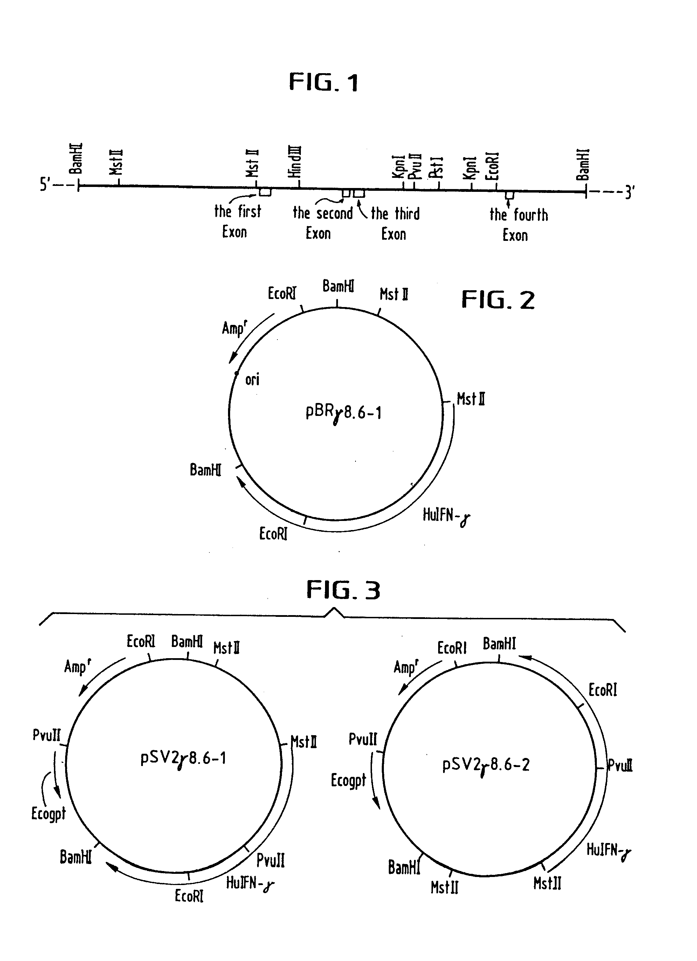

Removing intron sequences from the primary RNA transcribed from a gene is called splicing. The splicing is supposed to be a process necessary for the accumulation of stable mRNA or the transfer of mRNA from nucleus to cytoplasm. Therefore, it is supposed that the addition of viral introns to the intron-free cDNA sequence of HuIFN-γ leads to the intracellular accumulation of mRNA in the producing methods referred to above. As shown in Fig. 1, the HuIFN-γ gene is composed of four exons, three introns, and two sequences adjacent to the 5'-side and the 3'-side [Gray, P.W. et al. (1982): Nature, 298, 859; Taya, Y. et al. (1982): EMBO J., 1, 953). The present inventors presumed that mRNA which is transcribed from the original, intron-bearing, chromosomal DNA sequence of HuIFN-γ, and is submitted to splicing, is more stable and therefore is accumulated at higher concentration in the cytoplasm compared with mRNA which is transcribed from the cDNA sequence. A regulatory region which controls the expression of HuIFN-γ is present at the 5'-side of the HuIFN-γ gene sequence. This region which contains the DNA sequence to which the RNA polymerase binds and which initiates the synthesis of mRNA, is called promoter region. If the HuIFN-γ gene sequence which contains said promoter region is introduced into animal cells as it is, the production of HuIFN-γ is extremely low. This finding indicates that in order to obtain an effective production the 5'-promoter region of the HuIFN-γ gene which is in a repressed state in most animal cell cultures has to be replaced by other promoter regions which are able to initiate the synthesis of mRNA in animal cell cultures.

Whereas, in general, splicing of introns at correct sites is essential for the expression of normal and functional proteins, an unusual splicing was found when the insulin gene and the promoter region of SV40 were combined and introduced into COS cells [Laub, O. et al. (1983): J. Biol. Chem., 258, 6043]. As for expression of amylase, it is known that tissue-specific splicings take place and that by two different splicing processes, both salivary and hepatic amylases are synthesized from the same gene [Young, R.A. et al. (1981): Cell, 23, 451]. Also for adenovirus [Chow, L.T. (1977): Cell, 12, 1], a plural number of mRNAs and proteins were proved to be synthesized through different splicing processes from the same gene. Accordingly, some normal splicing processes are essential for producing the HuIFN-γ by introducing introncontaining HuIFN-γ genes into animal cell cultures.

HuIFN-γ is a glycoprotein. The structure of the sugar chain of HuIFN-γ is still unclear in many respects. For example, it is unknown whether there are differences between sugar chains of HuIFN-γ produced by cells of organisms other than human and HuIFN-γ produced in human cells in their structure and antigenic properties. The HuIFN-γ produced by human cells however is thought to be the same as the natural one, and to be higher in safety than those produced by cells of organisms other than human. The latter may cause problems such as allergic and shock reactions during the long-term therapeutic administration, since their preparations are possibly contaminated with components such as protein or the like or secretions from the parent organisms. In contrast, preparations of the IFN-γ produced by human cells never substantially contain any substance other than the human components, that is, substances intrinsically existing in the human blood. For these reasons, a high safety is expected for products derived from human cells.

For establishing human cell cultures producing HuIFN-γ, the cell can be tranformed by an HuIFN-γ expression vector. Such transformed cells, however, are killed by the self-produced HuIFN-γ since this generally has a strong toxicity against human cells. Therefore, it is extremely difficult to isolate transformed strains which can be subcultured. Thus, whereas transformed strains which are able to be subcultured and to produce HuIFN-γ can be easily obtained from hamster cells, transformed strains which are able to be subcultured are difficult to obtain from human cell cultures.

Most cells have a tendency to stick to vessel walls. Even if cultured under mechanical agitation of the culture medium, cells having such a tendency do not proliferate to separate off-springs, but yield cell floculations. The floculations are uneven in size, and result in an uneven cell density in the culture medium. In addition, because of difficulties in controlling the agitation conditions when the agitation is mild, cells exhibit a greater tendency to stick. If, on the other hand, the agitation is too strong the cells may be damaged.

Based on the above background, the Present inventors isolated chromosomal DNA coding for HuIFN-γ, and prepared plasmids in which the HuIFN-γ gene is ligated to a promoter sequence that functions in animal cells, for example, SV40 early promoter, SV40 late promoter, or thymidine kinase promoter of herpes simplex virus. Further these plasmids were attached to a selective marker gene, for example, Ecogpt. The resulting plasmids, for example, pSVeSmalγ, pSV2pTKγ, pSV2Lγ and pSV3Lγwere introduced into various animal cells according to the calcium phosphate method or the cell fusion method. It was found that in the obtained transformed cell lines the introduced human chromosomal DNA sequences were normally transcribed normally spliced and translated. Sugar chain-bearing active HuIFN-$\gamma$ was secreted. Furthermore, the inventors for the first time obtained subculturable transformed cell lines using HuIFN-$\gamma$-resistant cell cultures derived from humans, or HuIFN-$\gamma$-resistant mutants. It is thought that, basically, subculturable HuIFN-$\gamma$-producing cell lines can be derived from any sort of cell cultures derived from humans, and that, consequently natural-type HuIFN-$\gamma$ is high in safety.

In addition, the inventors succeeded in separating HuIFN-$\gamma$-producing strains by transformation of cell lines derived ,from blood cells. These are proliferable as separate cells which do not substantially stick to vessel walls during cultivation and hardly yield cell floculations. These HuIFN-$\gamma$-producing cell lines can be readily submitted to suspension culture processes for carrying out a large scale culture. The present invention provides a means for the large scale production of highly safe HuIFN-$\gamma$.

Figure 1 displays a restriction map of the 8.6Kb BamHI fragment which contains the HuIFN-$\gamma$ gene.

Figure 2 shows the plasmid pBR$\gamma$8.6-1.

Figure 3 shows the plasmids pSV2$\gamma$8.6-1 and pSV2$\gamma$8.6-2.

Figures 4(a) and 4(b) schematically show the construction of an expression vector, pSVeSmaI$\gamma$, capable of producing HuIFN-$\gamma$ in mammalian cell cultures.

Figs. 5-7 schematically show the construction of the expression vectors pSV2pTK$\gamma$, pSV2L$\gamma$ and pSV3L$\gamma$, respectively, which are capable of producing HuIFN-$\gamma$ in mammalian cell cultures.

Figures 8(a) and 8(b) are schematic outlines of a process for preparing the expression-vector pSVeBPV$\gamma$.

When human chromosomal DNA is cleaved with the restriction enzyme BamHI, the gene region which codes for HuIFN-$\gamma$ is contained within a DNA fragment of about 8.6kb, as shown in Fig. 1. Exons of HuIFN-$\gamma$ are present at four places separately. These exons on the DNA sequence are referred to as the first to fourth exon in order from the 5'-side, respectively (each indicated by a thick line in Fig. 1). On the sequences of the 5'-side adjacent to the first exon, there is a regulatory site which controls the expression of HuIFN-$\gamma$. The fourth exon has the signal AATAAA for polyadenylation of mRNA, following the termination codon. The first exon codes for a polypeptide which is composed of 38 amino acid residues. Twenty-three (23) N-terminal amino acids, which arise from the protein-synthesis-initiating amino acid, methionine,form a signal peptide, which is present at the N-terminus of many secretory proteins. The signal peptide is cleaved off during secretion from the cell. The chromosomal DNA sequence which codes for HuIFN-$\gamma$ is the DNA sequence which contains the 4 exons and 3 introns shown in Fig. 1. The chromosomal DNA sequence which codes for HuIFN-$\gamma$ is cloned from the human DNA. The human DNA is prepared by the method of Blin et al. [Blin, N. et al. (1976): Nucleic Acids. Res., 3, 2303] from human leukocytes or tissue, for example. As a vector for cloning the HuIFN-$\gamma$ gene, $\lambda$ phage vectors represented by Charon 28, plasmid vectors represented by pBR322, or cosmids represented by pHC79 are used. However, the gene manipulation technique is generally adopted with the use of the $\lambda$ phage vector with which large DNA fragments can be cloned with high efficiencies. That is, after cutting of human high-molecular weight DNA using an appropriate restriction enzyme, the DNA fragment is inserted instead of the substitutable region of $\lambda$ phage DNA for obtaining the recombinant phage DNA. Next, using the in vitro packaging technique, infectious phage particles are prepared. Then the phage particles are plated together with the host Escherichia coli to obtain plaques of recombinant phages [Enquist, L. et al. (1979): Methods in Enzymology, 68, 281; Horn, B. (1979): Methods in Enzymology, 68, 299]. For detection of the plaques of the recombinant phage which carries DNA fragments coding for HuIFN-$\gamma$, the plaque hybridization technique is used with 32p-labelled cDNA or synthetic DNA as a probe [Woo, S.L.C. (1979): Methods in Enzymology, 68, 389; Szostak, J.W. et al. (1979): Methods in Enzymology, 68, 419]. Furthermore, the recombinant phage which carries the HuIFN-$\gamma$ gene can be prepared in large amounts by recovering it from a plaque selected by the plaque hybridization, and by culturing it together with the host Escherichia coli. The recombinant phage DNA can be prepared according to the method described in Maniatis, T. et al. [(1982): Molecular Cloning, A Laboratory Mannual, Cold Spring Harbor Laboratory].

The amino acid sequence of HuIFN-$\gamma$ can be derived from the base sequence of the exon parts of the cloned HuIFN-$\gamma$ gene. The base sequence, in turn, can be determined according to the Maxam-Gilbert method [Maxam A.M. et al. (1980): Methods in Enzymology, 65, 499].

For the purpose of selective proliferation of the cells in which the desired gene is introduced and stably expressed, a DNA sequence composed of HuIFN-$\gamma$ sequence ligated to a functional promoter sequence, and a selectable marker gene, is appropriate. As the selectable marker in animal cells, such genes as Ecogpt [Mulligan, R.C. et al. (1980): Science, 209, 1422], neo [Southern, P.J. et al. (1982): J. Mol. Appl. Genet., 1, 327], and dhfr [Wigler, M. et al. (1980): Proc. Natl. Acad. Sci. USA, 77, 3567] are used.

As a promoter which functions in animal cell cultures, the SV40 early promoter is known. This promoter is contained by the Hind III-Pvu II fragment of SV40 DNA (about 350 base pairs in size) and has an activity as SV40 late gene promoter in the opposite orientation.

The thymidine kinase promoter of type 1 of herpes simplex virus (HSV) is a constitutive promoter similarly to SV40 early promoter. The structure has been shown by Wagner et al. [Wagner, M.J. et al. (1981): Proc. Natl. Acad. Sci. USA, 79, 1441.]. The functional promoter region contains the initiation origin of mRNA synthesis, but not the methionine codon which is the first amino acid of the protein that is regulated by said promoter.

For preparing a large amount of the DNA ( HuIFN-γexpression vector )sequence in which the sequence of the functional promoter region is ligated to the HuIFN-γ chromosomal DNA sequence,it is desirable that such a DNA is a plasmid or a phage DNA which can be replicated in Escherichia coli.

PlasmidspSVeSmalγ, pSV2pTKγ, and the like which are described in examples 4 to 8 meet the above conditions. In other words,any of these plasmids is characterized in that its DNA sequence comprises the origin of DNA replication (ori) of Escherichia coli and of the animal cell cultures, the HuIFN-γ chromosomal DNA sequence ligated to a functional promoter, and a selectable marker gene in Escherichia coli (Ampicillin resistance gene )and in animal cell cultures (Ecogpt).

For the introduction of DNA into animal cell cultures, such methods as the calcium phosphate method [Wigler,M.et al. (1977): Cell,11,223],microinjection method [Anderson,w.f. et al.(1980)- :Proc.Natl.Acad.Sci.USA, 77,5399], lyposome method,DEAE-dextran method or cell fusion method [Schoffner,W. et al. (1980) :Proc.Natl.Acad.Sci.USA,77,2163],are used, although these methods differ from each other in the transfection efficiency. As DNA material for the calcium phosphate method, microorganisms, as Escherichia coli, phages and DNA solutions can be used. In the cell fusion method, microbial protoplasts containing the desired DNA sequence as a plasmid are used.

It is known that the mRNA synthesis from the SV40 late promoter is increased in the presence of the SV40 T antigen (Keller, J.M. et al. (1984): Cell, 36, 381). It was found that the yield of HuIFN-γ was enhanced when cells were co-transfected with a DNA sequence which codes for the SV40 T antigen and the HuIFN-γ gene linked to the SV40 late promoter. When cells are co-transfected with a DNA sequence coding for SV40 T antigen, T antigen bearing plasmids such as pSV3gpt can be used. Further, when cells are transfected with the DNA sequence comprising the HuIFN-γ gene and the T antigen gene, both the HuIFN-γ and the T antigen gene are integrated into the cells more effectively than by co-transfection of them. It may also be possible to obtain cell lines highly expressing HuIFN-γ using various cell strains. Furthermore, it is also possible to use SV40 transformants as a host.

For the production of HuIFN-γ by cells into which a DNA sequence containing the chromosomal HuIFN-γ gene ligated to a functional promoter, is introduced, it is necessary that said DNA sequence is fitted for the RNA synthesis system inherent in the cells to be used, the maturation system of RNA, the protein synthesis system, the maturation system of protein, as well as for functions as secretion and the like. In addition for the synthesis of mRMA from the introduced DNA, it is necessary that capping at the 5'-end, splicing at normal sites, and polyadenylation of the 3'-end of the mRNA take place. Furthermore, for expression of active IFN, it is necessary that higher order structures of the IFN peptide to be synthesized are formed and maintained, that the signal peptide is precisely cleaved off and that the resulting peptide is secreted from the cell. The inventors investigated different animal cell strains available from the American type culture collection (ATCC). The cells were derived from hamster, monkey, mouse, rat or human, including CHO (hamster ovary cells), HeLa (human cerevicitis tumor cells), FL (human amnion cells), WISH (human amnion cells), Chimp liver (chimpanzee liver cells), WI-26VA4 (human lung cells transformed by SV40), and Vero (kidney cells of African green monkey). It is also possible to use other cells, hybridomas, normal and variant cells at least derived from vertebrates, and cells transformed by virus for the production of HuIFN-γ according to the present invention. Further improvements of safety of products from the cell lines transformed by SV40 are expected in comparison with the cell lines with an unaccountable malignancy provided that proper means are taken. As a transformant by SV40, WI-26 VA4 is known.

For selecting HuIFN-γ-resistant cell lines or HuIFN-γ-resistant variants out of many cell cultures derived from human subcultivation of culture cells in a medium containing HuIFN-γ by one unit to several ten thousand units is useful. Whereas HuIFN-γ-sensitive culture cells derived from human are denatured, deformed, and finally killed by the toxicity of HuIFN-γ during the subcultivation, the HuIFN-γ-resistant cell lines are not affected, but continue to grow. The latter therefore are easily selectable. In addition, HuIFN-γ-sensitive lines become significantly sensitive to a double-stranded RNA as poly I:C. When being treated with HuIFN-γ the cells are denatured markedly and killed when being cultured in a medium containing poly I:C. Thus the HuIFN-γ-resistant cell lines are distinguishable from these HuIFN-γ-sensitive cell lines.

Established cell lines of mammals are derived from blood cells as lymphoblasts, myeloblasts, monocytes and erythroblasts which are derived from their stem cells. Additionally cell lines were established from cells which are further differentiated or derived from said blastocytes. The established cells derived from blood cells which have been proliferated in a common medium display spherical morphology and are suitable for suspension cultures. Some of these cells, namely those producing globulin are advantageously used as hosts for the production of HuIFN-γ, because they have high ability to synthesize and secrete proteins.

The inventors investigated different animal cell cultures derived from blood cells, which were obtained from the ATCC, including MOPC-31C (plastocytoma_, MPC 11 (myeloma), MB III (lymphosarcoma), RBL-1 (leakemic basophilic granulocyte), RPMI 7666 (lymphoblastoid), MOLT-3 (acute lymphoblastic leukemia), L1210, CCRF-CEM, or EL4, U937, and X63-Ag8.653. They can produce HuIFN-γ active in established cells, hybridomas, normal and variant cells at least derived from mammalian blood cells, and cells transformed by virus, provided that the manufacturing methods described in the specifications of the invention are employed. It was found that cells which were obtained to produce HuIFN-γ by the introduction of HuIFN-γ chromosomal DNA sequence ligated to a functional promoter by, for example, the calcium phosphate method or the cell fusion method, can produce HuIFN-γ not only in a medium containing serum, but also in quite serum-free medium. The use of serum-free medium for production of HuIFN-γ makes the recovery and purification of HuIFN-γ from the medium easier.

Well-known methods are applicable for the recovery and purification of HuIFN-γ from medium. For example, IFN can be recovered by adsorption to controlled pore glass (Elecronucleonics Corp.), washing with PBS (0.15M NaCl-0.15M phosphoric acid buffer; pH = 7.4), elution with PBS containing polyethylene glycol by 50%, further adsorption to ConA-Sepharose, elution with phosphoric acid buffer containing 0.15M NaCl and 0.15M α-methyl-D-mannoside, and finally by passing it through Biogel-P-100.

The activity of IFN was determined with the CPE-inhibiting method with the combined use of FL cells and vascular stomatitis virus or sindovis virus [Philip, C. et al. (1981): Methods in Enzymology, 78, 387].

EXAMPLES

Various experiments which were relevant to the invention and described in the Examples given below, were carried out in accordance with "the Japanese Guideline on Recombinant DNA" prescribed by the Prime Minister. Detailed manipulations of phages, plasmids, DNA, various enzymes and Escherichia coli in Examples were performed by reference to the following jounals or books:

1. Proteins, Nucleic Acids and Enzymes, Vol. 26, No.4 (Extra Edition: Gene Manipulation) (1981). Kyroritsu Publishing Co.
2. Y. Takagi: Experimental Methods of Gene Manipulation, 1981. Kodansha Ltd.
3. Y. Takagi: Manual of Gene Manipulation, 1982. Kodansha Ltd.
4. T. Maniatis et al.: Molecular Cloning, A Laboratory Manual, 1982. Cold Spring Harbor Laboratory
5. L. Grossman et al.: Methods in Enzymology, Vol.65, 1980. Academic Press
6. R. Wu: Methods in Enzymology, Vol.68, 1979. Academic Press

Example 1

Cloning of HuIFN-γ gene

Blood samples were taken from several healthy adults, and, after collecting the buffy coat, hemolysed by adding about ten-fold volume of 0.83% $NH_4Cl$. The sample was washed with Eagle's MEM medium to obtain leucocytes. Ten billion ($10^{10}$) leucocytes were shaken with 50 ml of a solution containing 0.5M EDTA, 0.5% sarcosyl, and 100 μg/ml protease K for 3 hours at 50°C to be dissolved. The obtained solution was extracted with phenol three times, and the aqueous layer was dialysed against 20mM tris. HCl (pH 8.0), 10mM EDTA, and 10mM NaCl. The dialyzate was treated with 100 μg/ml ribonuclease for 3.5 hours at 37°C, again extracted with phenol, and dialysed against 20mM tris • HCl (pH 8.0), 1mM EDTA, and 10mM NaCl to obtain about 33 mg of high molecular human DNA. The human DNA was digested with the restriction enzyme BamHI, and then BamHI DNA fragments of about 8 to 9 kilobases (hereinafter abbreviated to kb) were prepared with sucrose density-gradient centrifugation. Lambda(λ)-phage vector charon 28A DNA (Bethesda Research Laboratories, Gaithersburg, USA) was digested with BamHI, and the fractions containing the left-arm and right-arm of Charon 28 were collected with sucrose density-gradient centrifugation, and recovered by ethanol precipitation: The DNA fragments with both arms of Charon 28 and the human BamHI fragments of 8 to 9 kb were ligated together using T4 DNA ligase. Then, the ligated DNA was submitted to an in vitro packaging technique according to the Enquist-Sternberg method [Enquist, L. and N. Sternberg (1979): Methods in Enzymol., 28, 281] to form plaques of recombinant phages with the use of Escherichia coli LE392 as a host. Next, HuIFN-γ gene-bearing recombinant phage clones were selected out according to the plaque hybridization technique [Benton, W.D. and R.W. Davis (1977): Sience, 196, 180]. Oligonucleotides CTTGGCTGTTAC, CCTGGCAGTAAC, and GCTCTTCGACCTCG were synthesized by the phosphotriester method [Miyoshi, K. et al. (1980): Nucleic Acids Res., 8, 5507], labelled with [$\gamma$-$^{32}$P]ATP and T4 polynucleotide kinase, and used as probes.

Four phage clones, S8-11 S18-6, S19-5 and S20-1 to be hybridized with all of used three synthetic DNA probes were obtained out of about two million recombinant phages. DNA was prepared from the respective phage clones, and digested with the restriction enzyme BamHI. As a result, DNA fragments of about 8.6 kb proved to be contained by each phage clone. Furthermore, by the restriction enzyme analysis, and the analysis of DNA sequences mentioned later, the selected four recombinant phage clones were identified as those containing HuIFN-$\gamma$ BamHI fragments of 8.6 kb.

Example 2

Preparation of pBR$\gamma$8.6-1, pSV2$\gamma$8.6-1 and pSV2$\gamma$8.6-2

DNA of phage clone S8-11 containing HuIFN-$\gamma$ gene was digested with the restriction enzyme BamHI, and ligated to a BamHI site of plasmid pBR322 which had been treated with bacterial alkaline phosphatase [Bolivar, F. et al. (1977): Gene, 2, 951 and transformed to Escherichia coli C600$\gamma^-$m$^-$. A transformant containing a plasmid into which the HuIFN-$\gamma$ gene of about 8.6 kb had been inserted into the BamHI site of pBR322 (ATCC31344) was selected out as transformants having ampicillin-resistant and tetracycline-sensitive characters This plasmid was designated as pBR$\gamma$8.6-1. The structure of pBR$\gamma$8.6-1 is shown in Fig. 2.

The plasmid pBR$\gamma$8.6-1 was digested with BamHI, DNA fragments of about 8.6 kb were prepared by agarose gel electrophoresis and ligated to the plasmid pSV2gpt (ATCC 37145) which had been treated with bacterial alkaline phosphatase with the use of T4DNA ligase and transformed to Escherichia coli C600$\gamma^-$m$^-$. Strains bearing the plasmids pSV2$\gamma$8.6-1 and pSV2$\gamma$8.6-2, whose structure are shown in Fig. 3, were selected from among other transformants.

plasmids pSV2$\gamma$8.6-1 and pSV2$\gamma$8.6-2 are plasmids into which the HuIFN-$\gamma$ gene-bearing DNA fragment has been inserted clockwise and counterclockwise into the BamHI sites of pSV2gpt, respectively. Respective plasmid DNA was prepared by cesium chloride density-equilibrium centrifugation. Plasmid DNA was prepared as needed with the use of Escherichia coli GM33 dam$^-$ as a host.

Example 3

Base sequence in HuIFN-$\gamma$ gene and amino acid sequence in HuIFN-$\gamma$

The base sequence in the HuIFN-$\gamma$ gene of plasmid pBR$\gamma$8.6-1 was determined according to the Maxam-Gilbert method [Maxam, A.M. and W. Gilber (1980): Methods in Enzymology, 65, 499]. The base sequences around four (4) exons coding for HuIFN-$\gamma$ were determined as follows:

5' – GATCAGCTTG

ATACAAGAACTACTGATTTCAACTTCTTTGGCTTAATTCTCTCGGAAACG

```
ATG AAA TAT ACA AGT TAT ATC TTG GCT TTT CAG CTC TGC
Met Lys Tyr Thr Ser Tyr Ile Leu Ala Phe Gln Leu Cys

ATC GTT TTG GGT TCT CTT GGC TGT TAC TGC CAG GAC CCA
Ile Val Leu Gly Ser Leu Gly Cys Tyr Cys Gln Asp Pro

TAT GTA AAA GAA GCA GAA AAC CTT AAG AAA TAT TTT GT
Tyr Val Lys Glu Ala Glu Asn Leu Lys Lys Tyr Phe
```

AAGTATGACTTTTTAATAGTACTTGTTTGTGGTTGAAAATGACTG......

.................................................. AG

```
AAT GCA GGT CAT TCA GAT GTA GCG GAT AAT GGA ACT CTT
Asn Ala Gly His Ser Asp Val Ala Asp Asn Gly Thr Leu

TTC TTA GGC ATT TTG AAG AAT TGG AAA GAG GTAAGCTGAA
Phe Leu Gly Ile Leu Lys Asn Trp Lys Glu
```

TATTCCCATTTGGCTAATTTTCCTGTTGCTTGCTTTCTGATGGATAAATT

CACATCATCCTCTGTTTGTGCTCTTTCCTTCCAAG | GAG AGT GAC AGA
                                      | Glu Ser Asp Arg

```
AAA ATA ATG CAG AGC CAA ATT GTC TCC TTT TAC TTC AAA
Lys Ile Met Gln Ser Gln Ile Val Ser Phe Tyr Phe Lys

CTT TTT AAA AAC TTT AAA GAT GAC CAG AGC ATC CAA AAG
Leu Phe Lys Asn Phe Lys Asp Asp Gln Ser Ile Gln Lys

AGT GTG GAG ACC ATC AAG GAA GAC ATG AAT GTC AAG TTT
Ser Val Glu Thr Ile Lys Glu Asp Met Asn Val Lys Phe

TTC AAT AGC AAC AAA AAG AAA CGA GAT GAC TTC GAA AAG
Phe Asn Ser Asn Lys Lys Lys Arg Asp Asp Phe Glu Lys

CTG ACT AAT TAT TCG GTGAGGCTATTTAAATTCTTTCTTTGGTTT
Leu Thr Asn Tyr Ser
```

CATTGCCGAGGGTCTTGCAAAGCATTTATTCTCCAGAAAGTAGACATTAG

CTATTTAACAGTTGCTAAAGCTATGAACTCAACTCATGGCTGAAACTCT-

. . . . . . . . . . . . . . . . . . . . . . . . . . . . . . . . . GTCCATTTTT

TAAAGGAATACTTTTATTTTCACTGACCATCATGACATTAGCAGAATATC

CTGATGGCTTATATGCCTGAAATTAATTTTGCTCTTTTCTTTCCCGATAG

```
GTA ACT GAC TTG AAT GTC CAA CGC AAA GCA ATA CAT GAA
Val Thr Asp Leu Asn Val Gln Arg Lys Ala Ile His Glu

CTC ATC CAA GTG ATG GCT GAA CTG TCG CCA GCA GCT AAA
Leu Ile Gln Val Met Ala Glu Leu Ser Pro Ala Ala Lys

ACA GGG AAG CGA AAA AGG AGT CAG ATG CTG TTT CGA GGT
Thr Gly Lys Arg Lys Arg Ser Gln Met Leu Phe Arg Gly

CGA AGA GCA TCC CAG TAA TGGTTGTCCTGCCTGCAATATTTGAA
Arg Arg Ala Ser Gln
```

TTTTAAATCTAAATCTATTTATTAATATTTAACATTATTTATATGGGGAA

TATATTTTTAGACTCATCAATCAAATAAGTATTTATAATAGCAACTTTTG

TGTAATGAAAATGAA

## Example 4

Preparation of pSVeSmaIγ and pSV2pTKγ

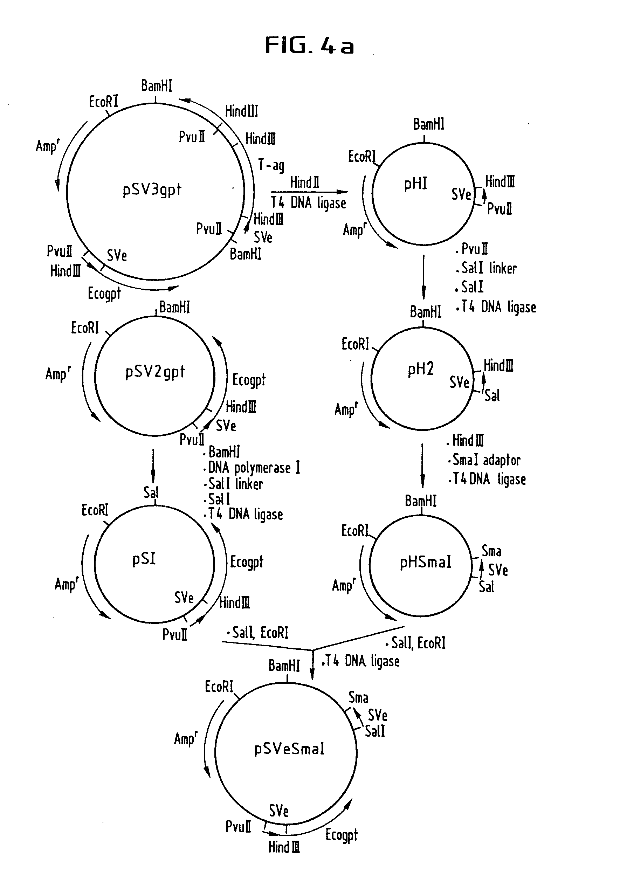

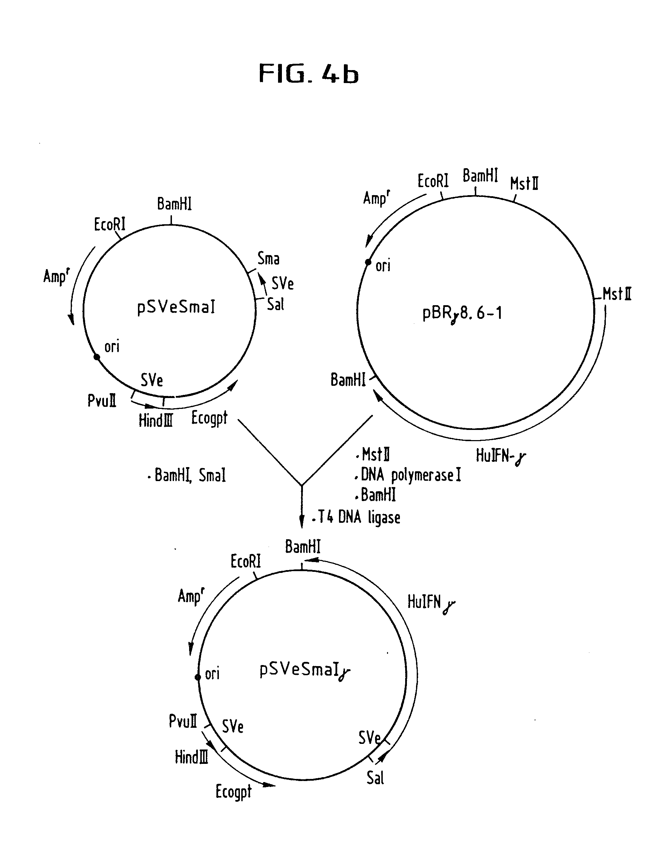

Plasmid pSVeSmalγ which has the sequence of SV40 promoter region and ligated chromosomal DNA sequence of HuIFN-γ, is prepared according to the procedures shown in Figs. 4-(a) and 4-(b), with the use of pBR8.6-1, pSV2gpt and pSV3gpt (ATCC37144) [Mulligan, R.C. and P. Berg (1980): Science, 209, 1422] as starting materials.

First, pSV3gpt (ATCC37144) was digested with Hind III, and the largest DNA fragment was made cyclic with T4 DNA ligase to make pH1. Next, the Pvu II site of pH1 was changed into the Sal I site with the use of Sal I linker to make pH2. Further, the Hind III site of pH2 was changed to the Sma I site with the use of Hind III - Sma I adaptor to make pHSmal. psv2gpt was digested with Bam HI, The termini were filled to make blunt ends with DNA polymerase I (klenow). A Sal I site was introduced with the use of Sal I linker and T4DNA ligase to make PSI. The PSI was digested with Sal I and EcoRI, and an ampicillin-resistance gene-bearing DNA fragment was ligated to the Sal I-EcoRI fragment of pHSmal having SV40 promoter region to make pSVeSmal. Next, pBRγ8.6-1 was digested with Mst II, and, after the termini were made blunt with DNA polymerase (Klenow), digested with BamHI to obtain HuIFN-γ sequence-bearing DNA fragment. The DNA fragment was introduced into pSVeSmal which was digested with Smal and BamHI, to make pSVeSmalγ.

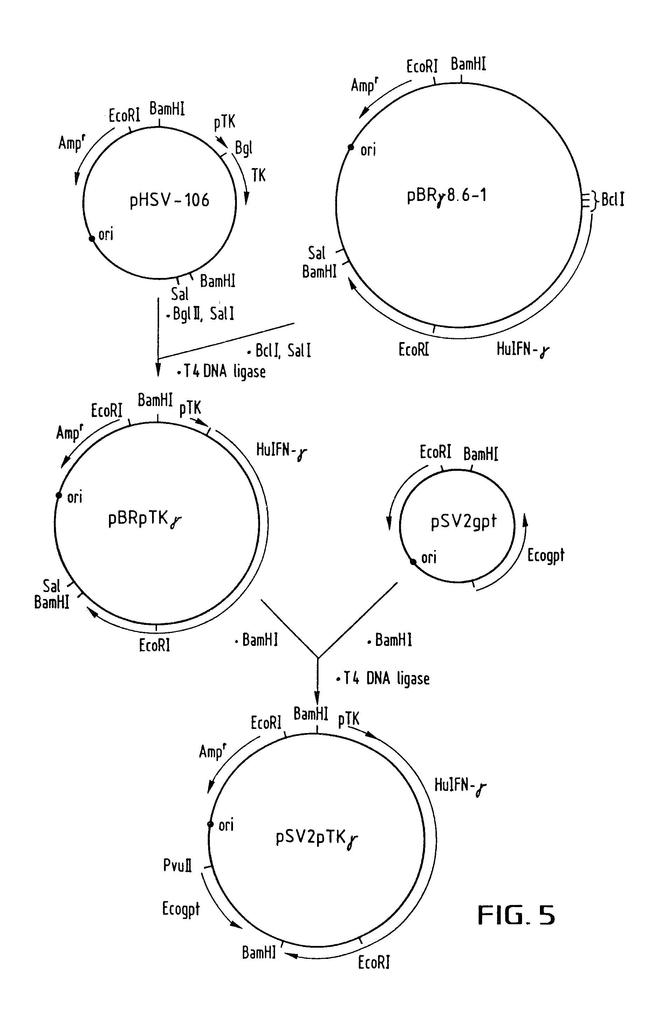

Plasmid pSV2pTKγ which has the sequence of thymidine kinase promoter region of herpes simplex virus type I and the HuIFN-γ gene, is prepared according to the procedures shown in Fig. 5, with the use of pBRγ8.6-1, pHSV106 (Bethesda Research Laboratories, Gaithersburg, USA) [Mcknight, S.L. and E.R. Gabis (1980): Nucleic Acids Res., 8, 5931], and pSVgpt as starting materials.

Sal I linker and Sma I adaptor used were d(pGGTCGACC) and d(pAGCTCCCGGG), respectively. Employed DNA polymerase I was Klenow fragment.

## Example 5

Preparation of pSV2Lγ

A HuIFN-γ-expression vector, pSV2Lγ was prepared according to the following procedures.

Plasmid pSV2gpt [Mulligan, R.C. and P. Berg (1980): Science, 209, 1422] having both ampicillin-resistance gene and guanine phosphoribosyl transferase gene (Ecogpt) of Escherichia coli, was digested with the restriction enzyme Pvu II. Bcl I linker (CTGATCAG) which had been phosphorylated with T4 polynucleotide kinase, was ligated to the termini with the use of T4 ligase.

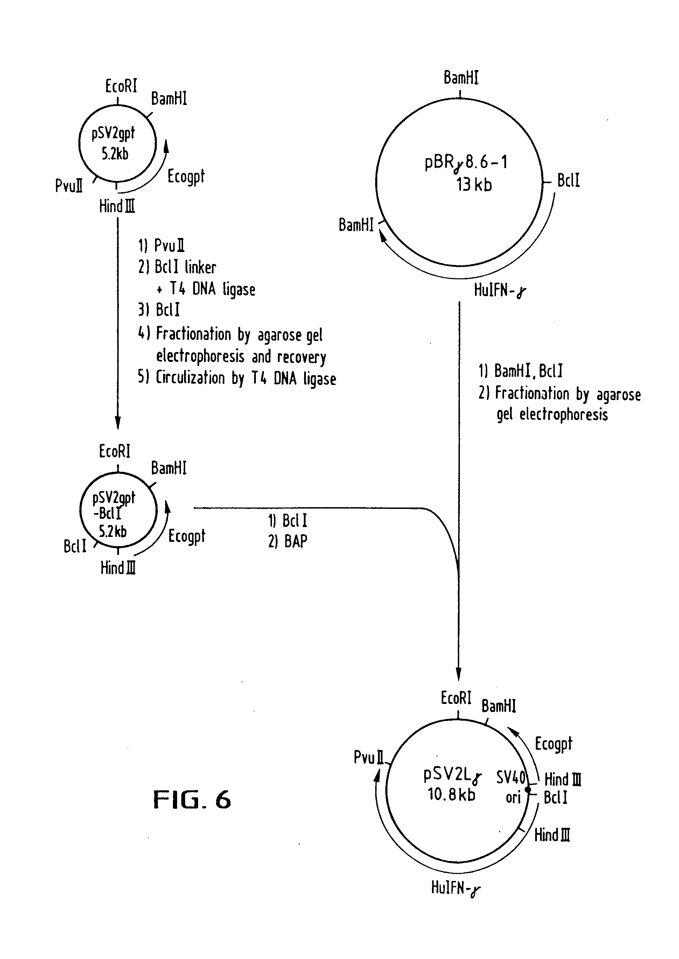

After treatment with the restriction enzyme Bcl I, the DNA fragment was recovered by 0.8% agarose gel electrophoresis. The recovered DNA fragment was circularized with the use of T4 DNA ligase to obtain pSV2gptBclI by transformation of E. coli GM33 dam⁻. The pBR 8.6-1 described in Example 2 was digested with the use of Bam HI and Bcl I. HuIFN-γ gene-bearing DNA fragment of 5.5 kb was recovered, and inserted into the Bcl I site of pSV2gptBclI to obtain pSV2Lγ. The preparation of pSV2Lγ is shown in Fig. 6.

## Example 6

Preparation of HuIFN-γ-expression vector pSV3Lγ

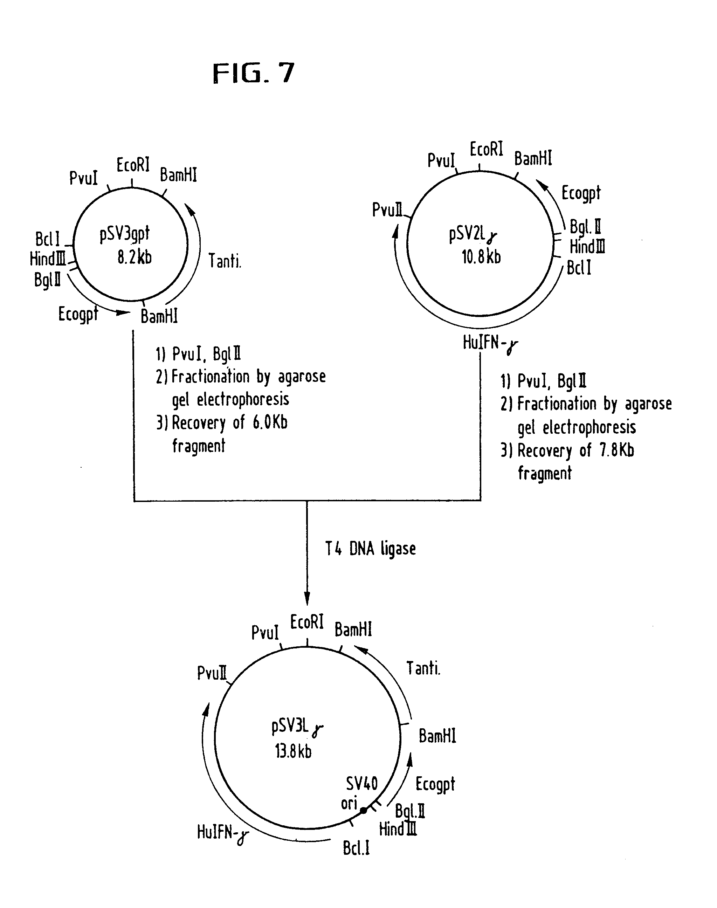

The plasmid pSV2Lγ described in Example 5 was digested with restriction enzymes Pvu I and Bgl II, and a HuIFN-γ gene-containing DNA fragment of about 7.8 kb was recovered by 0.8% agarose gel electrophoresis. The recovered fragment was ligated to another DNA fragment which contained SV40 T antigen of pSV3gpt [Mulligan, R.C. and P. Berg (1980): Science, 209, 1422] that had been digested with Pvu I and Bal II, and was circularized to pSV3Lγ. The preparation of pSV3Lγ is shown in Fig. 7.

## Example 7

Preparation of pSVeBPVγ

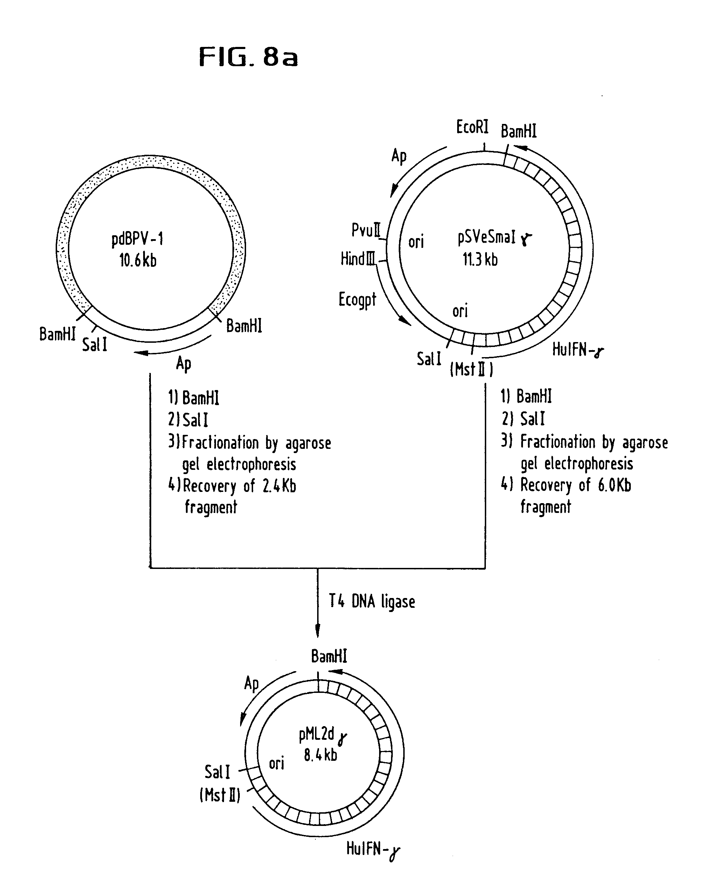

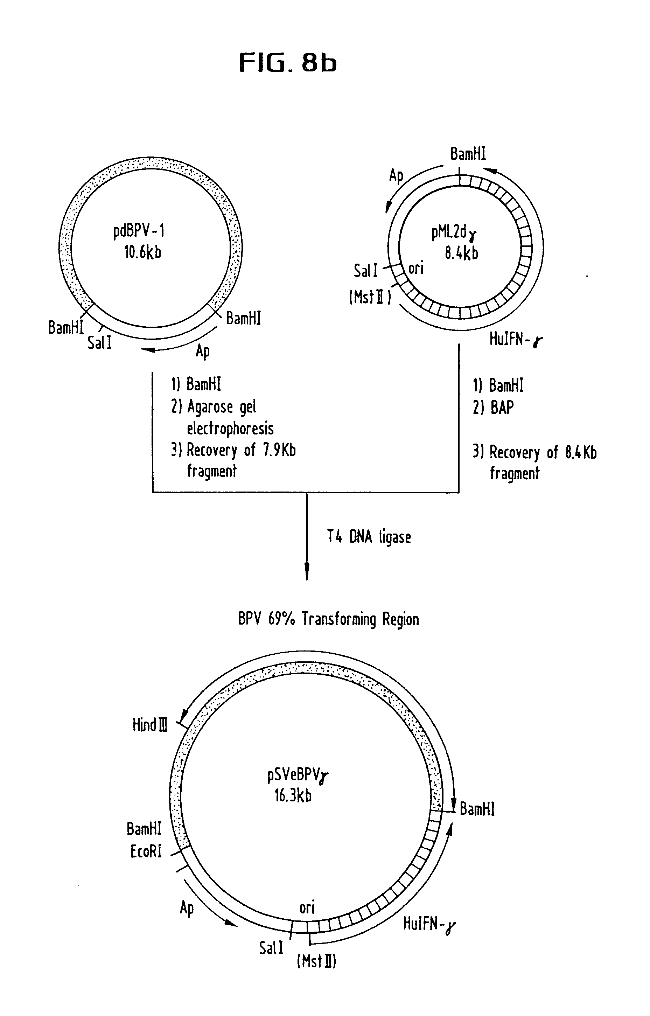

The plasmid pSVeSmalγ described in Example 4 was digested with the restriction enzymes Sal I and Bam HI to obtain a DNA fragment of about 6.0 kb which contained HuIFN-γ gene bearing SV40 early promotor. On the other hand, pdBPV-1 (ATCC 37134) [Sarver, N. et al. (1982): Proc. Natl. Acad. Sci. USA, 79, 7147] which was composed of both bovine papilloma virus genome and pML2d, was digested with Sal I and Bam HI to obtain an ampicillin-resistance gene-containing DNA fragment of about 2.4 kb. The obtained fragment of about 2.4 kb was ligated to the above fragment of about 6.0 kb to make pML2dγ. Next, the bovine papilloma virus genome-bearing DNA fragment of about 7.9 kb was cut out of pdBPV-1, and indroduced into the Bam HI site of pML2dγ to obtain plasmid pSVeBPVγ of about 16.3 kb. The preparation of pSVeBPVγ shown in Figs. 8-a and 8-b.

Example 8

Introduction of pSVeSmalγ and pSVpTKγ into animal cell cultures and production of HuIFN-γ

For the purpose to investigate the expression of HuIFN-γ gene of pSVeSmalγ or pSVpTKγ, the plasmid was introduced into varied animal cell cultures in accordance with procedures of Wigler et al. [Wigler et al. (1977): Cell, 11, 223]. The co-precipitate of the plasmid with calcium phosphate was added to cells ($3 \times 10^5$ cells/3.6-ml medium/6-cm round dish) which had been preliminarily grown in Eagle's MEM medium containing 10% fetal calf serum (hereinafter abbreviated as FCS). Cultivation was continued for 48 hours in the medium which was renewed 15 hours after the initiation. IFN activity involved in the respective medium was determined. As shown in the table below, expression was observed in every cell culture which contained pSVeSmal or pSV2pTKγ, while little expression was observed in cell cultures which contained pSV2 8.6-1, pBRγ8.6-1, pSV5gpt or calf thymus DNA.

| Introduced plasmid (7.2μg/dish) | IFN (units/ml) Cell culture | | | | | |
|---|---|---|---|---|---|---|
| | CHO-Kl (CCL-61) | Chimp. Liver | Hela (CCL-2) | FL (CCL-62) | WISH (CCL-25) | WI-26VA4 (CCL-95.1) |
| pSVeSmalγ | 64 | 32 | 64 | 2 | 1 | 32 |
| pSV2pTKγ | 16 | 16 | 16 | 1 | 1 | 32 |
| pSV2γ8.6-1 | 0.12 | ND | 0.23 | ND | ND | ND |
| pBRγ8.6-1 | ND | ND | ND | ND | ND | ND |
| pSV5gpt | ND | ND | ND | ND | ND | ND |

Note: ND referred to undetectable, but less than 0.06. The CCL numbers refer to ATCC depositions .

Example 9

Production of HuIFN-γ in normal media and serum-free media

The medium of CHO cells into which pSVeSmalγ or pSV2pTKγ had been introduced in Example 8, was replaced by an MEM medium which contains 10% FCS, 25 μg/ml mycophenolic acid, and 250 μg/ml xanthine. Then, mycophenolic acid-resistant strains were separated therefrom, respectively. Each mycophenolic acid-resistant strain was grown confluently in 24-well multidish, then cultured for 24 hours with 5% FCS-containing MEM medium, and with quite FCS-free MEM medium, respectively, and the IFN activities of the respective media were determined. Isolated cell lines produced IFN regardless of the existence of serum or not as follows:

12

### Production of IFN by pSVeSmaIγ-introduced CHO cells

| Medium | IFN activity (unit/ml/24 hrs) Mycophenolic acid-resistant CHO cell line | | | | |
|---|---|---|---|---|---|
| | CHO-21 | CHO-29 | CHO-46 | CHO-76 | CHO-92 |
| 5%FCS, 95%MEM | 450 | 450 | 117 | 3750 | 234 |
| 100%MEM | 281 | 141 | 281 | 1126 | 70 |

### Production of IFN by pSV2pTKγ-introduced CHO cells

| Medium | IFN activity (unit/ml/24 hrs) Mycophenolic acid-resistant CHO cell line | | | | |
|---|---|---|---|---|---|
| | TK-12 | TK-13 | TK-15 | TK-18 | TK-23 |
| 5%FCS 95%MEM | 12 | 96 | 192 | 192 | 6 |
| 100% MEM | 4 | 32 | 64 | 64 | 2 |

Example 10

Purification and properties of HuIFN-γ

Mycophenolic acid-resistant CHO cells, CHO-33, were isolated by the procedures mentioned in Example 9, and cultured in an MEM medium to obtain 120 ml of culture liquid. Then, 1.0 g of Controlled Pore Glass CPG350 (Electronucleonics Corp.; mesh size 120/200) was added to 60 ml of the obtained culture liquid, agitated for 3 hours at 4°C, packed into a column, washed with 150mM-phosphate-0.15M NaCℓ buffer (pH 7.4), and eluted by the same buffer which, in addition, contained 50% ethylene glycol. The active fraction was diluted 10-fold with 20mM phosphate buffer, passed through a 5-ml ConA-Sepharose Column to get IFN adsorbed. Then the adsorbed IFN was eluted with 20mM phosphate buffer containing 0.15M NaCℓ and 0.15M α-methyl-D-mannoside. The active fractions were collected, dialyzed against 0.2M ammonium acetate (pH 6.0) and 0.15M NaCℓ, concentrated by polyethylene glycol 20000, and passed through Biogel P-100 (2.6 x 60cm) which had been equilibrated with the same buffer to obtain an IFN sample.

The obtained IFN sample lost its activity on treating with acid of pH 2.0 or with 0.1% SDS. In testing the sensitivity-promoting effect of the purified IFN on the cytotoxicity of poly I:C, the promoting effect was observed only for human cells. In fact, cells were grown in monolayer on a 24-hole multidish, treated with IFN at 300 units/ml for 20 hrs, and then cultured for 24 hours in an MEM medium containing 10 μg/ml poly I:C. While CHO and BHK (hamster cells) underwent no changes, cells of FL, WISH and Hela were observed to become round and exfoliated.

Example 11

Introduction of pSV2Lγ and pSV3Lγ into animal cell cultures, and production of HuIFN-γ

For the purpose to investigate the expression of HuIFN-$\gamma$ gene contained in pSV2L$\gamma$ and pSV3L$\gamma$, the plasmid was introduced into animal cell cultures in accordance with the procedures of Wigler et al. [Wigler et al. (1977): Cell, 11, 223]. The co-precipitate of the plasmid with calcium phosphate was added to cells ($3 \times 10^5$ cells/3.6 ml medium/6cm-diameter dish) which had been preliminarily grown in an Eagle's MEM medium containing 10% newborn calf serum. The medium was renewed 15 hours after the initiation, and still incubated for 48 hours. Then, IFN activity involved in the medium was determined. As shown below, the expression of IFN activity was observed in the cell strains of CHO-KI, Vero or WI-26VA4 which contained pSV2L$\gamma$ or pSV3L$\gamma$, while no activity was observed in the cell strains which contained only calf thymus DNA.

| Introduced DNA (7.2µg/dish) | IFN activity (units/ml) Cell lines | | |
|---|---|---|---|
| | CHO-Kl | Vero | WI-26 VA4 |
| pSV2L$\gamma$ | 1.8 | 1.8 | 64 |
| pSV3L$\gamma$ | 28 | 28 | 128 |
| pSV2L$\gamma$ + pSV3gpt | 28 | 14 | 64 |
| pSV3L$\gamma$ + pSV3gpt | 28 | 28 | 128 |
| pSV3gpt | 0 | 0 | 0 |
| Calf thymus DNA | 0 | 0 | 0 |

Further, the concurrent introduction of pSV2L$\gamma$ and pSV3gpt, containing the SV40 T antigen-coding gene, exhibited a 8 to 15 times higher activity than the single introduction of pSV2L$\gamma$. WI-26 VA4 continuously expresses SV40 T antigen, and exhibites higher expression than CHO-KI.

Example 12

Production of HuIFN-$\gamma$ in normal media and serum-free media

The medium of CHO-KI cells into which pSV3L$\gamma$ had been introduced in Example 11, was replaced by MEM medium which contained 10% FCS, 25 $\mu$g/ml mycophenolic acid, and 250 $\mu$g/ml xanthine. Then, mycophenolic acid-resistant cells were isolated therefrom, respectively. Mycophenolic acid-resistant cells were grown confluently in 24-well multidish, then cultured for 24 hours with 5% FCS-containing MEM medium, and with quite FCS-free MEM medium, respectively, and the IFN activities involved in respective media were determined. Five (5) cells out of separated 35 cells secreted 188 units/ml. or more IFN into the medium including the one secreting more than 1500 units/ml. By cultivating the latter cell in a FCS-free medium, 150 units/ml IFN-containing culture medium was obtained.

Example 13

Purification of HuIFN-$\gamma$

HuIFN-$\gamma$-yielding CHO cells obtained in Example 12 were cultured for 4 days in a 5% FCS-containing MEM medium, and 60 ml of the medium was recovered therefrom. To the recovered culture medium, 0.5 g of Controlled Pore Glass (Electronucleonics Corp.) CPG350 (meshsize 120/200) was added, agitated for 3 hours at 4°C, filled into a column, washed with 150mM phosphate buffer (pH 7.4) and 0.15M NaCℓ, and eluted with the same buffer containing 50% ethylene glycol. Then the obtained active fractions were diluted 10-fold with 20mM phosphate buffer. Thus, about 20 000 units of IFN was purified and recovered.

Example 14

Introduction of pSVeBPV into animal cell cultures,and yield of HuIFN-γ

First,7.2μg of PSVeSPV was introduced into CHO-kl cells and mouse cells C127l in accordance with the method of Wigler et al. [Wigler et al.(1977):Cell,11,223]. The co-precipitate of the plasmid with calcium phosphate was added to cells(3 to 10x10⁵ cells/3.6ml medium/6cm-diameter dish) which had been preliminarily incubated in a Dulbecco modified Eagle's MEM medium (hereinafter referred to as DMEM medium) containing 10% FCS. After 15 hours the medium was renewed,and after 48 hours an aliquot of the medium was recovered,and its IFN activity was determined as 24units/ml for CHO-Kl,and 14units/ml for C-127.

In another way,a strain transformed through 2-week cultivation was sub-planted to a 96-well multidish at a rate of 200 μl medium/well,and cultured for 3 days.As a result, transformed strains which had activities of 750 units/ml for CHO-Kl,and 1500 units/ml for c-127,respectively,were obtained.

Example 15

Selection of HuIFN-γ resistant strains

Cell cultures of human origin such as HeLa,FL,WISH, and WI-26 VA-4, were cultured for 24 hours using a 24-well multidish plate in an MEM medium which contained 100 units/ml HuIFN-γ and 5% FCS. After removing the culture liquid, the cultured cells were washed with PBS one time,added to with MEM buffer containig 10 μg/ml poly I:C,and further cultivated at 37°C. After 16 to 24 hours,it was observed that cells of HeLa, FL and WISH were got round and exfoliated from the plate bottom,but cells of WI-26 VA4 were not. This finding suggests that WI-26 VA4 cells are resistant to HuIFN-γ.

Example 16

Separation of HuIFN-γ-resistant mutants

FL cells were cultured on the whole bottom surface of 75 cm² of a flask. Then the culture was renewed by an MEM medium which contained 100 units/ml HuIFN-γ, and 10 μg/ml poly I:C, and still incubated for 48-hours. Although most of FL cells were exfoliated from the bottom, the medium was replaced by another MEM medium containing 100 units/ml HuIFN-γ, and still incubated for about one month. Colonies of HuIFN-γ-resistant FL cells were developed, proliferated, and established.

Example 17

Preparation of transformed cell lines

The pSVeSmalγ or pSV2pTKγ which had been prepared so as to express HuIFN-γ in Example 4, or the pSV2gpt which had no HuIFN-γ gene, was introduced into the HuIFN-γ-sensitive HeLa, FL or WISH cells, or the HuIFN-γ-resistant WI-26VA4 cells, and the HuIFN-γ-resistant FL cells in accordance to the calcium phosphate method [Wigler et al. (1977): Cell, 11, 223]. The co-precipitate of 1,44 μg plasmid with calcium phosphate was added to the above cells (3×10⁵ cells/3.6 ml medium/6cm-diameter disk)which had been preliminarily grown in an Eagle's MEM medium containing 10% newborn calf serum. After 15 hours, the medium was renewed, and after 48-hour incubation, the medium was replaced by another medium which contained 25 μg/ml mycophenolic acid, 250 μg/ml xanthine, 0.1 μg/ml aminopterin, 25 μg/ml adenine, and 5 μg/ml thymidine, and after further 2-week incubation the numbers of developed transformant colonies were counted. As shown below, transformants were obtained from all used strains for pSV2gpt, while few transformant colonies were obtained from HuINF-γ-sensitive strains for HuIFN-γ gene-containing pSVeSmalγ or pSV2pTKγ, although many were from HuIFN-γ-resistant WI-26 VA4 and HuIFN-γ-resistant FL cells.

| Plasmid | Numbers of transformant colonies formed | | | | |
|---------|------|----|------|---------|------------------|
| | HeLa | FL | WISH | WI-26VA4 | HuIFN-resistant FL |
| pSV2gpt | 120 | 52 | 28 | 36 | 40 |
| pSVeSmal$\gamma$ | 1 | 0 | 3 | 28 | 37 |
| pSV2pTK$\gamma$ | 1 | 1 | 0 | 25 | 44 |

Example 18

Production of HuIFN-$\gamma$ by WI-26 VA4

The colonies of WI-26 VA4 transformant obtained in Example 17 using pSveSmal$\gamma$ or pSV2pTK$\gamma$ were separated, and proliferated in an MEM medium containing 5% FCS. The transformant was proliferated on the whole bottom surface of the 24-well multiplate. After the medium was renewed, and incubated for 24 hours, the IFN activity in the culture liquid was determined. The IFN activities of 32.3 and 524 units/24 hr/$10^6$ cells were observed for transformants by pSVeSmal$\gamma$ and pSV2pTK$\gamma$, respectively. In addition, using 1.44 $\mu$g of pSV2$\gamma$8.6-1, pSV2pTK$\gamma$ or pSVeSmal$\gamma$, WI-26 VA4 strain was transformed in a similar way to that in Example 17. The obtained results are as follows.

| Plasmid | Number of transformed cell IFN-$\gamma$ activity (Unit/24hr/$10^6$cells) | | | | Total transformed cells |
|---------|---|-----|--------|--------|---------|
| | 0 | <10 | <$10^2$ | <$10^3$ | |
| pSV2$\gamma$8.6-1 | 8 | 1 | 0 | 0 | 9 |
| pSV2pTK$\gamma$ | 4 | 0 | 0 | 1 | 5 |
| pSVeSmaI$\gamma$ | 5 | 1 | 2 | 0 | 8 |

Example 19

Production of HuIFN-$\gamma$ by HuIFN-$\gamma$-resistant FL cells

The colonies of HuIFN-$\gamma$-resistant FL transformant obtained in Example 17 using pSV2pTK$\gamma$ were separated, and proliferated in an MEM medium containing 5% FCS. The transformant was proliferated on the whole bottom surface of the 24-well multiplate. After the medium was renewed, and incubated for 24 hours, the IFN activity in the culture liquid was determined. The development of the activity of 3 units/24 hr/$10^6$ cells was observed.

Example 20

Expression of IFN by blood cell-derived cell lines

The pSVeSmal$\gamma$ described in Example 4 was introduced into blood cell-derived cell lines obtained from ATCC or others according to the method of Oi, et al. [Oi, V.T. et al. (1980): Proc. Natl. Acad. Sci. USA, 80, 825]. Plasmid pSVeSmal$\gamma$-bearing Escherichia coli K-12 strain C600$\gamma^-$m$^-$ was cultured with an L medium at 37°C until the absorbance at 600 nm reached 0.6 to 0.8. After the addition of chloramphenicol up to 125 $\mu$g/ml and the further culturing for 12 to 16 hours, the bacteria were centrifuged and collected. Then, cold 0.05M tris. HCl (pH 8.0) which contained sucrose by 20% per 250 ml medium, was added to disperse the bacteria. After the addition of 0.25 ml of 5 mg/ml lysozyme solution, and 5-minute standing on ice, 0.5 ml of 0.25M EDTA (pH 8.0) was added, and the liquid was allowed to stand again for 5 minutes on ice. Then, after 0.5 ml of 0.05M Tris•HCℓ (pH 8.0) was added again, the liquid was incubated for 10 to 15 minutes at 37°C to protoplastize the bacteria. The protoplast was diluted with 10 ml of DMEM medium containing 10% sucrose, and allowed to stand for 10 minutes at room temperature. The cells were cultured in DMEM medium containing 10% FCS to $10^6$ cells/ml, added to by protoplast suspension by 5 ml per 2 x $10^6$ cells, and centrifuged at about 500xg at room temperature. After removing the supernatant, the precipitate was added to by 2 ml of 50% polyethylene glycol solution (pH 8.0) in DMEM medium, gently stirred, and centrifuged for 3 minutes at 500xg. The cells were washed with 7 ml of DMEM medium, dispersed into DMEM medium containing 10% FCS, and cultured at a cell concentration of 2 x $10^5$ cells/well•ml with a 24-well multidish plate. After a 24-hour cultivation, the culture supernatant was taken, and its IFN activity was determined. As shown below, pSVeSmalγ-introduced cells exhibited IFN activity.

| Production of IFN by blood cell-derived cell lines | | |
|---|---|---|
| Cell line | | IFN activity(units/ml/48hrs) |
| MOPC-31C | (CCL-130) | 160 |
| MPC 11 | (CCL-167) | 30 |
| MB III | (CCL-32) | 3 |
| RBL-1 | (CRL-1378) | 3 |
| RPMI7666 | (CCL-114) | 3 |
| MOLT-3 | (CRL-1552) | 3 |
| L1210 | (CCL-219) | 60 |
| CCRF-CEM | (CCL-119) | 12 |
| *EL-4 | | 60 |
| U937 | (CRL-1593) | 12 |
| X63-Ag8 | (CRL-1580) | 60 |

*EL-4: Farrar, J.J. et al.: (1980) J. Imm. 125, 2555.
Note: The CCL and CRL numbers refer to ATCC depositions.

Example 21

Production of HuIFN-γ with normal media and serum-free media

The medium of MOPC-31C cells into which pSVeSmalγ had been introduced by the protoplast fusion method in Example 20, was replaced by a DMEM medium which contained 10% FCS, 25 μg/ml mycophenolic acid, 250 μg/ml xanthine, 5 μg/ml thymidine, 0.1 μg/ml aminopterin, and 25 μg/ml adenine, and further cultured for 3 to 4 weeks, and mycophenolic acid-resistant cell colonies were isolated therefrom. Each of the mycophenolic acid-resistant cells Was cultured on the whole bottom surface of a 24-well multidish, and further cultured with 5% FCS-containing or FCS-free DMEM medium for 48 hours, and the IFN activity in the medium was determined. As shown below, the isolated cell lines proved to produce IFN regardless of the presence or absence of serum. Similarly, both MPC 11 and EL4 transformed lines produced IFN.

Production of IFN by isolated mycophenolic resistant cell lines

| Medium | IFN activity (units/ml/48hrs) Origin of mycophenolic acid resistant strain | | |
|---|---|---|---|
| | MOPC-31C | MPC11 | EL4 |
| 5%FCS, 95%DMEM | 1500 | 80 | 80 |
| 100%DMEM | 200 | ND* | ND* |

*ND: Not tested.

**Claims**

1. A DNA sequence containing
   (a) a chromosomal DNA sequence coding for human interferon-γ (HuIFN-γ) ligated to a DNA sequence of the promoter region of a gene that functions in an animal cell; and
   (b) a selectable marker gene for an animal cell.

2. The DNA sequence according to claim 1, wherein said promoter region is a promoter region of a gene displaying constitutive expression.

3. The DNA sequence according to claim 2, wherein said promoter region is the early promoter region of simian virus 40 (SV40), or the promoter region of the thymidine kinase gene of herpes simplex virus (HSV-1).

4. The DNA sequence according to claim 2, wherein said promoter region is the late promoter region of SV40.

5. The DNA sequence according to claim 4, wherein said promoter region is present in said DNA sequence in combination with a T antigen gene sequence of SV40.

6. The DNA sequence according to any one of claims 1 to 5, additionally containing:
   (c) a selectable marker in a microorganism; and
   (d) an origin for the replication of said DNA sequence in a microorganism.

7. The DNA sequence according to any one of claims 1 to 6 which is pSVeSmalγ, having the restriction map shown in Figure 4b, pBRpTKγ, having the restriction map shown in Figure 5, pSV2pTKγ, having the restriction map shown in Figure 5, pSV2Lγ, having the restriction map shown in Figure 7, PSV3Lγ, having the restriction map shown in Figure 7.

8. An animal cell transformed with a DNA sequence according to any one of claims 1 to 7.

9. The animal cell according to claim 8, which is of vertebrate origin.

10. The animal cell according to claim 8 or 9, which is of mammalian origin.

11. The animal cell according to any one of claims 8 to 10 which is of human origin.

**12.** The animal cell according to claim 11, which is HuIFN-γ-resistant.

**13.** The animal cell according to claim 11, which is a HuIFN-γ-resistant mutant.

**14.** The animal cell according to any one of claims 8 to 13, which is transformed with SV40.

**15.** The animal cell according to any one of claims 8 to 14, which is derived from a blood cell-derived cell line.

**16.** The animal cell according to claim 15, which is derived from a lymphatic cell line.

**17.** The animal cell according to claim 15, which is derived from a globulin-producing cell line.

**18.** The animal cell according to any one of claims 8 to 17, which is a CHO, HeLa, FL, WISH, WI-26 VA4, MOPC-31C, MPC 11, EL4, MBIII, RPMI 7666, MOLT-3, RBL-1, or L1210 cell, or a derivative thereof.

**19.** A method for preparing HuIFN-γ, comprising the steps of (i) cultivating an animal cell according to any one of claims 8 to 18, and (ii) recovering the produced HuIFN-γ.

**20.** The method according to claim 19, wherein said cultivation in step (i) is effected in a culture medium and wherein said HuIFN-γ is recovered from the culture medium.

**21.** The method according to claim 19, wherein said method comprises the steps of (i) proliferating said animal cell in animal bodies, and (ii) recovering HuIFN-γ from said animal bodies.

**22.** The method according to any one of claims 19 and 20, wherein said methods comprise the steps of (i) proliferating said animal cell in animal bodies, (ii) recovering said animal cells from said animal bodies, and (iii) cultivating said animal cell in a culture medium.

**23.** The method according to any one of claims 20 to 22, wherein said culture medium is a serum-free culture medium.

**24.** A microorganism containing a DNA sequence according to any one of claims 1 to 7.

**Patentansprüche**

**1.** DNA-Sequenz, enthaltend
(a) eine menschliches Interferon-γ (HuIFN-γ) codierende chromosomale DNA-Sequenz, ligiert mit einer DNA-Sequenz der Promotor-Region eines in einer tierischen Zelle funktionierenden Gens; und
(b) ein selektierbares Markergen für eine tierische Zelle.

**2.** DNA-Sequenz nach Anspruch 1, wobei die Promotor-Region die Promotor-Region eines Gens ist, das eine konstitutive Expression aufweist.

**3.** DNA-Sequenz nach Anspruch 2, wobei die Promotor-Region die frühe Promotor-Region von Simian Virus 40 (SV40) oder die Promotor-Region des Thymidinkinasegens von Herpes simplex-Virus (HSV-1) ist.

**4.** DNA-Sequenz nach Anspruch 2, wobei die Promotor-Region die späte Promotor-Region von SV40 ist.

**5.** DNA-Sequenz nach Anspruch 4, wobei die Promotor-Region in der DNA-Sequenz in Kombination mit einer T-Antigen-Gensequenz von SV40 vorkommt.

**6.** DNA-Sequenz nach einem der Ansprüche 1 bis 5, zusätzlich enthaltend:
(c) einen in einem Mikroorganismus selektierbaren Marker; und
(d) einen Replikations-Ursprung der DNA-Sequenz in einem Mikroorganismus.

**7.** DNA-Sequenz nach einem der Ansprüche 1 bis 6, die pSVeSmalγ mit der in Figur 4b dargestellten Restriktionskarte, pBRpTKγ mit der in Figur 5 dargestellten Restriktionskarte, pSV2pTKγ mit der in Figur 5 dargestellten Restriktionskarte, pSV2Lγ mit der in Figur 7 dargestellten Restriktionskarte oder pSV3Lγ mit der in Fig. 7 dargestellten Restriktionskarte ist.

**8.** Tierische Zelle, transformiert mit einer DNA-Sequenz nach einem der Ansprüche 1 bis 7.

**9.** Tierische Zelle nach Anspruch 8, die Vertebraten-Ursprungs ist.

**10.** Tierische Zelle nach Anspruch 8 oder 9, die Säuger-Ursprungs ist.

**11.** Tierische Zelle nach einem der Ansprüche 8 bis 10, die menschlichen Ursprungs ist.

**12.** Tierische Zelle nach Anspruch 11, die HuIFN-γ-resistent ist.

**13.** Tierische Zelle nach Anspruch 11, die eine HuIFN-γ-resistente Mutante ist.

**14.** Tierische Zelle nach einem der Ansprüche 8 bis 13, die mit SV40 transformiert ist.

**15.** Tierische Zelle nach einem der Ansprüche 8 bis 14, die aus einer aus einer Blutzelle stammenden Zellinie stammt.

**16.** Tierische Zelle nach Anspruch 15, die aus einer lymphatischen Zellinie stammt.

**17.** Tierische Zelle nach Anspruch 15, die aus einer Globulin herstellenden Zellinie stammt.

**18.** Tierische Zelle nach einem der Ansprüche 8 bis 17, die eine CHO-, HeLa-, FL, WISH-, WI-26 VA4-, MOPC-31C-, MPC 11-, EL4-, MBIII-, RPMI 7666-, MOLT-3-, RBL-1-, oder L1210-Zelle oder ein Abkömmling davon ist.

**19.** Verfahren zur Herstellung von HuIFN-γ, umfassend die Schritte der
   (i) Züchtung einer tierischen Zelle nach einem der Ansprüche 8 bis 18; und
   (ii) Gewinnung des hergestellten HuIFN-γ.

**20.** Verfahren nach Anspruch 19, wobei die Züchtung in Schritt (i) in einem Kulturmedium vollzogen wird und HuIFN-γ aus dem Kulturmedium gewonnen wird.

**21.** Verfahren nach Anspruch 19, wobei das Verfahren die Schritte der
   (i) Vermehrung der tierischen Zelle in Tierkörpern; und
   (ii) Gewinnung von HuIFN-γ aus den Tierkörpern umfaßt.

**22.** Verfahren nach einem der Ansprüche 19 und 20, wobei die Verfahren die Schritte der
   (i) Vermehrung der tierischen Zelle in Tierkörpern;
   (ii) Gewinnung der tierischen Zellen aus den Tierkörpern; und
   (iii) Züchtung der tierischen Zelle in einem Kulturmedium umfaßt.

**23.** Verfahren nach einem der Ansprüche 20 bis 22, wobei das Kulturmedium ein serumfreies Kulturmedium ist.

**24.** Mikroorganismus, enthaltend eine DNA-Sequenz nach einem der Ansprüche 1 bis 7.

**Revendications**

**1.** Une séquence d'ADN contenant
   (a) une séquence d'ADN chromosomique codant pour l'interféron-γ humain (HuIFN-γ) lié à une séquence d'ADN de la région du promoteur d'un gène qui fonctionne dans une cellule animale; et
   (b) un gène marqueur permettant la sélection, pour une cellule animale.

**2.** La séquence d'ADN selon la revendication 1, dans laquelle ladite région du promoteur est une région

20

du promoteur d'un gène présentant une expression constitutive.

3. La séquence d'ADN selon la revendication 2, dans laquelle ladite région du promoteur est la région du promoteur précoce du virus simien 40 (SV 40), ou la région du promoteur du gène de la thymidine kinase du virus herpès simplex (HSV-1).

4. La séquence d'ADN selon la revendication 2, dans laquelle ladite région du promoteur est la région du promoteur tardif de SV 40.

5. La séquence d'ADN selon la revendication 4, dans laquelle ladite région du promoteur est présente dans ladite séquence d'ADN en combinaison avec une séquence du gène de l'antigène T de SV 40.

6. La séquence d'ADN selon l'une quelconque des revendications 1 à 5, contenant de plus:
   (c) un marqueur permettant la sélection dans un microorganisme; et
   (d) une origine pour la réplication de ladite séquence d'ADN dans un microorganisme.

7. La séquence d'ADN selon l'une quelconque des revendications 1 à 6 qui est pSVeSmaI-$\gamma$, ayant la carte de restriction montrée dans la figure 4b, pBRpTK-$\gamma$, ayant la carte de restriction montrée dans la figure 5, pSV2pTK-$\gamma$, ayant la carte de restriction montrée dans la figure 5, pSV2L-$\gamma$, ayant la carte de restriction montrée dans la figure 7, pSV3L-$\gamma$, ayant la carte de restriction montrée dans la figure 7.

8. Une cellule animale transformée par une séquence d'ADN selon l'une quelconque des revendications 1 à 7.

9. La cellule animale selon la revendication 8, qui a pour origine un vertébré.

10. La cellule animale selon la revendication 8 ou 9, qui a pour origine un mammifère.

11. La cellule animale selon l'une quelconque des revendications 8 à 10 qui est d'origine humaine.

12. La cellule animale selon la revendication 11, qui est résistance à HuIFN-$\gamma$.

13. La cellule animale selon la revendication 11, qui est un mutant résistant à HuIFN-$\gamma$.

14. La cellule animale selon l'une quelconque des revendications 8 à 13, qui est transformée par SV 40.

15. La cellule animale selon l'une quelconque des revendications 8 à 14, qui est dérivée d'une lignée cellulaire dérivée de cellule sanguine.

16. La cellule animale selon la revendication 15, qui est dérivée d'une lignée cellulaire lymphatique.

17. La cellule animale selon la revendication 15, qui est dérivée d'une lignée cellulaire productrice de globuline.

18. La cellule animale selon l'une quelconque des revendications 8 à 17, qui est une cellulule CHO, HeLa, FL, WISH, WI-26 VA4, MOPC-31C, MPC 11, EL4, MBIII, RPMI 7666, MOLT-3, RBL-1, L1210, ou une cellule dérivée de l'une d'elles.

19. Une méthode pour préparer HuIFN-$\gamma$, comprenant les étapes de (i) cultiver une cellule animale selon l'une quelconque des revendications 8 à 18, et (ii) isoler le HuIFN-$\gamma$ produit.

20. La méthode selon la revendication 19, dans laquelle ladite culture de l'étape (i) est effectuée dans un milieu de culture et dans laquelle ledit HuIFN-$\gamma$ est isolé du milieu de culture.

21. La méthode selon la revendication 19, dans laquelle ladite méthode comprend les étapes de (i) faire proliférer ladite cellule animale dans des corps animaux, et (ii) isoler HuIFN-$\gamma$ desdits corps animaux.

22. La méthode selon l'une quelconque des revendications 19 et 20, dans laquelle lesdites méthodes comprennent les étapes de (i) faire proliférer ladite cellule animale dans des corps animaux, (ii) isoler lesdites cellules animales desdits corps animaux, et (iii) cultiver ladite cellule animale dans un milieu de culture.

23. La méthode selon l'une quelconque des revendications 20 à 22, dans laquelle ledit milieu de culture est un milieu de culture sans sérum.

24. Un microorganisme contenant une séquence d'ADN selon l'une quelconque des revendications 1 à 7.

# FIG. 1

# FIG. 2

# FIG. 3

23

EP 0 167 852 B1

FIG. 4a

24

# FIG. 4b

FIG. 5

FIG. 6

# FIG. 7

# FIG. 8a

# FIG. 8b

pdBPV-1
10.6kb

BamHI
SalI
BamHI
Ap

pML2dγ
8.4kb

BamHI
Ap
SalI
(MstⅡ)
HuIFN-γ

1) BamHI
2) Agarose gel
electrophoresis
3) Recovery of 7.9Kb
fragment

1) BamHI
2) BAP
3) Recovery of 8.4Kb
fragment

T4 DNA ligase

BPV 69% Transforming Region

pSVeBPVγ
16.3kb

HindⅢ
BamHI
EcoRI
Ap
ori
SalI
(MstⅡ)
BamHI
HuIFN-γ